(19) Europäisches Patentamt
European Patent Office
Office européen des brevets

(11) Publication number: 0 086 543 B1

# (12) EUROPEAN PATENT SPECIFICATION

(45) Date of publication of patent specification: 07.05.86

(51) Int. Cl.$^4$: B 01 J 29/04, B 01 J 37/30

(21) Application number: 83200221.6

(22) Date of filing: 12.02.83

## (54) Crystalline silicate catalysts with deactivated external surface, and process for its deactivation.

(30) Priority: 17.02.82 NL 8200615

(43) Date of publication of application:
24.08.83 Bulletin 83/34

(45) Publication of the grant of the patent:
07.05.86 Bulletin 86/19

(84) Designated Contracting States:
BE DE FR GB IT NL

(56) References cited:
EP-A-0 005 315
DD-A- 111 091
GB-A-2 019 394
US-A-3 404 192
US-A-3 702 886
US-A-4 254 297
US-A-4 273 753

(73) Proprietor: STAMICARBON B.V.
Mijnweg 1
NL-6167 AC Geleen (NL)

(72) Inventor: Frenken, Petrus Mathias Gerardus
Cunegondisstraat 29
NL-6017 ED Thorn (NL)

(74) Representative: Leherte, Georges Maurice
Lucien Marie et al
Octrooibureau DSM P.O.Box 9
NL-6160 MA Geleen (NL)

EP 0 086 543 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for deactivating catalytically active sites on the external surface of crystalline alumino and/or borosilicate catalysts.

Aluminosilicates, respectively borosilicates, constitute crystalline modifications of silicon dioxide, in the crystal lattice of which silicon dioxide silicon atoms have been replaced by aluminium, respectively boron, atoms. The Si/Al, respectively Si/B, ratio of these aluminosilicates, respectively borosilicates, may vary within very wide limits, but qualifying for the use as silicate catalysts are mainly those aluminosilicates, respectively borosilicates, for which the Si/Al, respectively Si/B, ratios are between 6, respectively 3, and 2000. Such aluminosilicates, respectively borosilicates, will hereinafter be referred to by the term zeolites, respectively boralites.

Therein silicon can be replaced by germanium. In the lattice minor quantities of the silicon and/or germanium can have been replaced by iron, chromium, vanadium, molybdenum, arsenic, manganese or gallium.

The internal pore system of zeolites and boralites is determined by the crystal structure of the relative silicates.

Zeolites are in themselves generally known from literature, for instance from the US—A—3702886 in particular, while boralites were described in the US—A—4269813 and 4254297.

In the preparation of zeolites and boralites the silica-alumina ratio, respectively the silica-boronoxide ratio, is essentially determined by the specific materials and the relative quantities of these materials used in the preparation of the zeolite, respectively the boralite. The catalytically active sites will occur not only in the pores of the silicate crystallite but also on the external surface. These catalytically active sites, of course, also occur near the entrances to the pores. As a result, when these silicates are used as catalysts in, for instance, the conversion of hydrocarbons, carbon will be formed, in the long run, at the entrances of the pores, so that the pores will be closed or be less accessible in course of time to the substances to be converted or to the product formed. In consequence hereof the catalytic properties of the catalyst decrease and the carbon formed will have to be removed. This is laborious and expensive while, moreover, the yield of product converted strongly decreases with the passage of time.

From the DD—A—111 091 patent it is known to block the active sites on the external surfaces of zeolite catalysts with alkaline compounds the molecular size of which exceeds that of the pores of the zeolite.

From the US—A—3,404,192 it is known to poison the external surfaces of zeolite catalysts with substances not capable of penetrating into the pores of the zeolite.

From the US—A—4,273,753 it is known to treat zeolites with dealuminating agents containing halogen. If the molecular size of the dealuminating agent is greater than the pore openings, substantially the external surfaces of the zeolites will be dealuminated.

The disadvantage of these known processes is that, in order to avoid the partial deactivation of the pores themselves, there must be a substantial difference in size between the molecules of the reagents used for deactivating the external surface and the openings of the pores.

Moreover the processes described above are limited to the treatment of catalysts having relatively small pores.

The object of the invention is to provide a more universal and efficient process for deactivating at least part of catalytically active sites on the external surface of crystalline silicates, in which process a stable catalyst having a high selectivity and a long life is obtained.

According to the invention this is achieved by making the silicate pores inaccessible to a deactivating agent and by subsequently contacting the silicate with the deactivating agent. The silicate pores are made inaccessible to the deactivating agent by saturating the pore volume of the silicate with an organic component that does not form a solution or mixture with the deactivating agent.

The organic component may be, for instance, hydrocarbons such as in particular aromatic or alkylaromatic hydrocarbons. Very suitable for this purpose is toluene.

Suitable deactivating agents are solutions of alkali metal salts in a polar solvent, preferably water. Preference is given to using sodium as alkali metal. Selective deactivation of the external surface of the silicate is effected by cation exchange of active protons for relatively inactive $Na^+$ cations.

The reaction schemes of this cation exchange on the surface of a zeolite, respectively boralite, are shown in scheme 1, respectively 2, of the sheet of drawings.

The process according to the invention is elucidated diagrammatically by means of scheme 3 of the sheet of drawings. I represents the state of the silicate as started from. Near the entrances to the pores there are active $H^+$ sites which must be exchanged for inactive $Na^+$ sites. II represents the state in which the silicate pore volume is saturated with an organic component such as, for instance, toluene. III represents the state after cation exchange of active protons ($H^+$) for inactive $Na^+$ cations on the external surface. IV represents the state after drying of the silicate, the organic component having been expelled from the pore system. A catalyst has then been obtained the external surface of which is selectively deactivated.

The catalytically active sites on the external surface of the silicate have preferably at least partly been replaced by catalytically inactive sodium sites. In the process according to the invention the silicate is contacted with the deactivating agent at a temperature of between 0 and 200°C for at least half an hour. The process of cation exchange can be carried out according to processes known per se, consequently

according to both a static and a dynamic process. The process of filling of the silicate pore volume, too, can be carried out according to any process known per se.

The invention will now be elucidated by means of examples without, however, being limited to the modes of realization described here.

Example I

Application, as catalyst, of a zeolite treated according to the present invention

a. In the preparation of a zeolite catalyst according to the invention 4 g ZSM 5 zeolite in acid form (HZSM-5 prepared according to the general process of Example 3 of the US patent 3,702,886) was put in a horizontal flow reactor and washed with air at 500°C for 16 hours. The zeolite was subsequently contacted with toluene until the zeolite particles tended to stick together. After that the zeolite was treated with a 0.2 M NaCl solution in water. Subsequently the zeolite was air-dried at 120°C for 16 hours and subsequently dried with air at 500°C for 16 hours. The pores were now entirely free of toluene.

b. 10 parts by weight of the zeolite thus prepared were diluted also with 90 parts by weight of Aerosil OX-50 (Registered Trade Mark) (high purity amorphous silica with specific surface area of 50 $m^2/g$, marketed by Degussa) and used as catalyst for the conversion of butylene-1 at a temperature of 600°C and a butylene-1/nitrogen molar ratio of 1 and a space velocity

$$(\text{WHSV}=\frac{\text{feed butylene}}{\text{quantity active catalyst}}\,\frac{\text{g/hour}}{\text{g}})\text{ of }1.4\text{ hour}^{-1}.$$

With the zeolite treated according to the invention the conversion continued to be more than 90% for 30 hours. In the same span of time the selectivity of the zeolite treated according to the present invention only fell from 48% to 30% as regards the conversion of butylene-1 to aromatic compounds.

Comparative Example

Application, as catalyst, of a zeolite according to US patent 3,702,886

10 parts by weight of the product obtained according to US patent 3,702,886 were diluted with 90 parts by weight of Aerosil OX-50 (Registered Trade Mark) (Degussa) and used as catalyst for the conversion of butylene-1 under the same circumstances as described in Example Ib. The conversion fell to 93% within 30 hours. In the same span of time the selectivity fell from 44% to 13% as regards the conversion of butylene-1 to aromatic compounds.

Example II

Application of a boralite, treated according to the invention, as catalyst for the rearrangement of cyclohexanone-oxime to ε-caprolactam

4 g boralite prepared according to the US patent No. 4,254,297 was treated as described in Example Ia.

A gas mixture of cyclohexanoneoxime, toluene, $CO_2$ and water in a molar ratio of 1:3:7:1 were passed over the boralite treated in this manner.

The temperature was 340°C.

The space velocity

$$(\text{WHSV}=\frac{\text{feed cyclohexanoneoxime}}{\text{quantity active catalyst}}\,\frac{\text{g/h}}{\text{g}})\ 0.8\text{ hour}^{-1}.$$

The conversion of the cyclohexanoneoxime and the selectivity to ε-caprolactam as functions of time are shown in the following table.

| t (hours) | 1 | 2 | 3 | 4 | 5 | 15 |
|---|---|---|---|---|---|---|
| conversion (%) | 100 | 100 | 100 | 100 | 100 | 100 |
| selectivity ε-caprolactam | 58 | 58 | 58 | 58 | 58 | 58 |

Comparative Example

Application of the boralite obtained according to US 4,254,297 as catalyst for the rearrangement of cyclohexanone-oxime to ε-caprolactam

The catalyst obtained according to US patent 4,254,297 was used for the rearrangement of cyclohexanone-oxime to ε-caprolactam under the circumstances described in Example II.

The result is shown in the table below.

| t (hours) | 1 | 2 | 3 | 4 | 5 |
|---|---|---|---|---|---|
| conversion (%) | 100 | 99.4 | 98.2 | 96.1 | 93.4 |
| selectivity ε-caprolactam | 58 | 58 | 58 | 58 | 58 |

**Claims**

1. Process for deactivating at least part of catalytically active sites on the external surfaces of crystalline aluminosilicate and/or borosilicate catalysts, characterized in that the pores of the silicate are made inaccessible to a deactivating agent and the silicate is subsequently contacted with the deactivating agent.

2. Process according to claim 1, characterized in that the pores of the silicate are made inaccessible to the deactivating agent by saturating the pore volume of the silicate with an organic component that cannot mix with the deactivating agent or cannot dissolve in it.

3. Process according to claim 2, characterized in that the organic component is an aromatic or alkylaromatic hydrocarbon.

4. Process according to claims 2 and 3, characterized in that the organic component is toluene.

5. Process according to claims 1—4, characterized in that the deactivating agent is a solution of an alkali metal salt in water.

6. Process according to claims 1—5, characterized in that the alkali metal is sodium.

7. Process according to claims 1—6, characterized in that the silicate is contacted with the deactivating agent at a temperature of between 0—200°C.

8. Process according to claims 1—7, characterized in that the silicate is contacted with the deactivating agent for at least half an hour.

**Patentansprüche**

1. Verfahren zur Desaktivierung mindestens eines Teils der katalytisch aktiven Stellen an den Außenoberflächen von kristallinen Aluminosilicat- und/oder Borosilikatkatalysatoren, dadurch gekennzeichnet, daß die Poren des Silicats unzugänglich für ein Desaktivierungsmittel gemacht werden und das Silicat anschließend mit dem Desaktivierungsmittel in Kontakt gebracht wird.

2. Verfahren nach Anspruch 1, dadurch gekennzeichnet, daß die Poren des Silicats unzugänglich für das Desaktivierungsmittel gemacht werden, indem das Porenvolumen des Silicats mit einer organischen Komponente gesättigt wird, die sich mit dem Desaktivierungsmittel nicht vermischen oder sich nicht darin auflösen kann.

3. Verfahren nach Anspruch 2, dadurch gekennzeichnet, daß die organische Komponente ein aromatischer oder alkylaromatischer Kohlenwasserstoff ist.

4. Verfahren nach den Ansprüchen 2 und 3, dadurch gekennzeichnet, daß die organische Komponente Toluol ist.

5. Verfahren nach den Ansprüchen 1—4, dadurch gekennzeichnet, daß das Desaktivierungsmittel eine Lösung eines Alkalimetallsalzes in Wasser ist.

6. Verfahren nach den Ansprüchen 1—5, dadurch gekennzeichnet, daß das Alkalimetall Natrium ist.

7. Verfahren nach den Ansprüchen 1—6, dadurch gekennzeichnet, daß das Silicat mit dem Desaktivierungsmittel bei einer Temperatur zwischen 0—200°C in Kontakt gebracht wird.

8. Verfahren nach den Ansprüchen 1—7, dadurch gekennzeichnet, daß das Silicat mit dem Desaktivierungsmittel während mindestens einer halben Stunde in Kontakt gebracht wird.

**Revendications**

1. Procédé de désactivation d'au moins une partie des sites à activité catalytique situés sur les surfaces externes de catalyseurs cristallins aux aluminosilicates et/ou aux borosilicates, caractérisé en ce que les pores du silicate sont rendus inaccessibles à un agent de désactivation et que le silicate est ensuite mis en contact avec l'agent de désactivation.

2. Procédé selon la revendication 1, caractérisé en ce que les pores du silicate sont rendus inaccessibles à l'agent de désactivation par saturation du volume poreux du silicate avec un composant organique incapable de se mélanger avec l'agent de désactivation et incapable de s'y dissoudre.

3. Procédé selon la revendication 2, caractérisé en ce que le composé organique est un hydrocarbure aromatique ou alkylaromatique.

4. Procédé selon les revendications 2 et 3, caractérisé en ce que le composant organique est le toluène.

5. Procédé selon les revendications 1 à 4, caractérisé en ce que l'agent de désactivation est une solution d'un sel de métal alcalin dans l'eau.

6. Procédé selon les revendications 1 à 5, caractérisé en ce que le métal alcalin est le sodium.

7. Procédé selon les revendications 1 à 6, caractérisé en ce que le silicate est mis en contact avec l'agent de désactivation à une température comprise entre 0 et 200°C.

8. Procédé selon les revendications 1 à 7, caractérisé en ce que le silicate est mis en contact avec l'agent de désactivation pendant au moins une demi-heure.

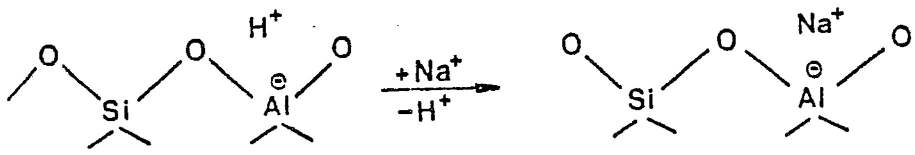


Scheme 1

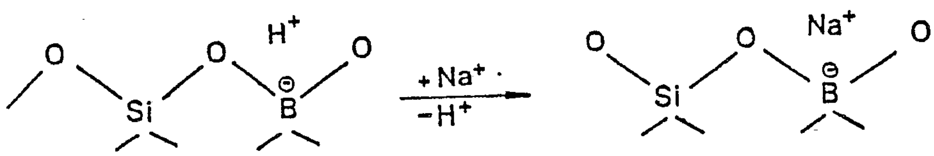


Scheme 2

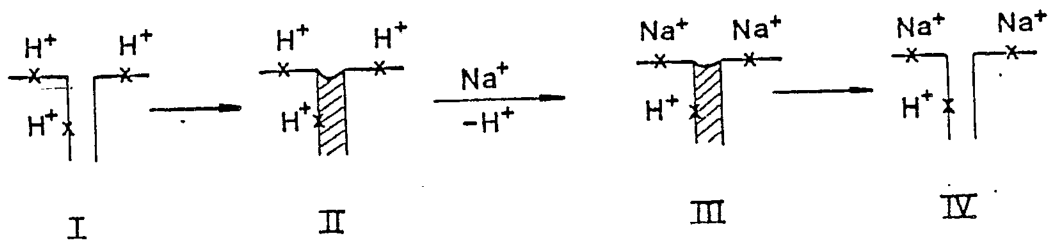


Scheme 3